# EUROPEAN PATENT SPECIFICATION

(11) **EP 2 367 446 B1**
(45) Date of publication and mention of the grant of the patent: **04.12.2019**
(21) Application number: 09836940.8
(22) Date of filing: 17.12.2009
(51) Int. Cl.: A23L 7/10, A23L 27/12, A23L 27/00, A23L 29/30, A61K 36/899, A61K 36/54

(54) **FOOD-BASED SUPPLEMENT DELIVERY SYSTEM**
ABGABESYSTEM FÜR NAHRUNGSERGÄNZUNGEN
SYSTÈME D'ADMINISTRATION DE COMPLÉMENT ALIMENTAIRE

(30) Priority: 17.12.2008 US 337371; 16.12.2009 US 639882
(43) Date of publication of application: 28.09.2011
(73) Proprietor: Gorris, Mark, Saint Louis, MO 63131 (US)
(72) Inventor: Gorris, Mark, Saint Louis, MO 63131 (US)
(74) Representative: Casbon, Paul Richard
(86) International application number: PCT/US2009/068447
(87) International publication number: WO 2010/078023

(56) References cited:
- US-A- 6 051 236
- US-A- 6 051 236
- US-A1- 2004 265 472
- US-A1- 2005 048 020
- US-A1- 2005 249 859
- US-A1- 2006 172 053
- US-A1- 2008 085 343
- US-A1- 2008 175 928
- US-A1- 2008 233 245
- HLEBOWICZ JOANNA ET AL: "Effect of cinnamon on postprandial blood glucose, gastric emptying, and satiety in healthy subjects", THE AMERICAN JOURNAL OF CLINICAL NUTRITION, AMERICAN SOCIETY FOR NUTRITION, US, vol. 85, no. 6, 1 June 2007 (2007-06-01), pages 1552-1556, XP002609982, ISSN: 0002-9165
- JOANNA HLEBOWICZ ET AL: 'Effect of cinnamon on postprandial blood glucose, gastric emptying, and satiety in healthy subjects' AMERICAN JOURNAL OF CLINICAL NUTRITION vol. 85, no. 6, June 2007, pages 1552 - 1556, XP002609982

## Description

### BACKGROUND OF THE INVENTION

### 1. Field of the Invention

The present invention relates to an edible composition of matter. This disclosure also relates to the field of delivery mechanisms for supplements, such as nutritional supplements or other materials thought to have medicinal value, which delivery mechanisms comprise placing the supplement in a food-based substrate which supplement is designed to enhance the substrate.

### 2. Description of Related Art

It is an old adage in medicine that the only difference between a medicine and a poison is in the amount consumed. Recent medical science continues to confirm this general proposition. Certain materials when consumed in relatively small quantities are "medicinal" while consumption in larger quantities can be detrimental (e.g. red wine). Alternatively, certain materials which are commonly present in food (e.g. vitamins) when provided in significantly greater quantities than is normally present in food can provide increased health benefits.

In the Am. J. Nutr. 2007 85:1552-6, American Society of Nutrition, "Effect of cinnamon on postprnadial blood glucose, gastric emptying, and satiety in healthy subjects J. Hlebowicz, et. al., it has been shown that the intake of cinnamon reduces blood glucose level and delayed gastric emptying.

One such material where the latter appears to be true is cinnamon. Cinnamon is believed to act as an appetite suppressant as well as providing other health benefits when taken in a sufficient dose. Cinnamon is also currently used in a variety of food items such as cereals, breads, candies, and cookies as a flavoring. However, the inclusion of cinnamon in these foods as a flavoring is in a relatively small dose. While there may be some limited health benefit from ingestion of this cinnamon, flavoring, and not health benefits, is the primary purpose of its inclusion in these products and in most the percentage of cinnamon is insufficient to provide for any meaningful benefit without undue consumption of the food item.

In the utilization of cinnamon for health benefit, the cinnamon is generally separated from a foodstuff and provided in a more "medicinal" format such as a tablet. This is because the amount of cinnamon needed for health benefit is generally significantly more than would be used in a recipe. It appears that, to provide for medicinal benefit, cinnamon should be taken at a rate of 1 gram per day or greater. Typical dosage is then provided as two one-half gram doses, or one full gram dose, via a commercial capsule or tablet. These supplements are designed to be swallowed because they generally contain concentrations of cinnamon far in excess of the amounts used for flavorings. Such a dose is thought to act as an appetite suppressant as well as providing for other health related benefits specifically related to digestion. These benefits include, but are not limited to, improved glucose metabolism and improved blood sugar maintenance. Such a concentrated amount, if it was to be used as a flavoring, would often be overpowering for many traditional cinnamon flavored food items and give them an unpleasant taste with a heavy bitterness or tartness. It could also cause a burning sensation or choking response.

Cinnamon's use as a flavoring is well understood and while this can provide for a pleasant taste, the amount used for flavoring is generally in quantities either so limited as to not have a significant medicinal benefit when a normal amount of the foodstuff is consumed (for example one or two standard servings), or, if the foodstuff was consumed in greater amounts, the negative impact from consuming such a large quantity of the other materials in the foodstuff would generally counterbalance the medicinal benefit of the consumption of cinnamon and may introduce other health risks such as those present from consumption of an overabundance of calories and/or fat compared to the recommended daily intake for those items.

Many of the items made with cinnamon as a flavoring are also sweet and usually relatively high in calories. As cinnamon alone is generally a bitter taste, a sweet taste is often a good balance. As much of the sweetness is provided by refined sugars, eating large quantities of these foods will generally serve to counteract some or all of the cinnamon benefits or possibly pose undesirable health issues (including dental issues) as well. This is especially true if the product containing the cinnamon were to be consumed on a regular/frequent basis (e.g. daily or more than once daily), as would be necessary to gain some medicinal benefit from the cinnamon.

As one ready example of the benefits of cinnamon consumption, the consumption of cinnamon is believed to have appetite suppressant effects and assist in controlling blood sugar if consumed in amounts above 1 gram per dose. However, a foodstuff using cinnamon as a flavoring generally includes only a small percentage of cinnamon and also includes a relatively large amount of fats, sugars and other undesirable food elements which would counteract the nutritional benefit as an appetite suppressant and increase blood sugar by adding additional sugar to the diet. Consumption of such foodstuffs to gain the benefit of an appetite suppressant would therefore be generally contraindicated. Therefore, these products do not serve as an effective method for cinnamon delivery.

There has recently been a push to provide for supplements in a more consumable as opposed to medicational form. Children's chewable vitamins are a basic example of these which have existed for many years. Specifically, they have provided the supplement (in this case a vitamin supplement) in a form which is designed to be eaten and has an acceptable taste as opposed to simply being swallowed. This form is still, however, in many respects medicational. The product is not really a consumable "food" but is simply a tablet in a chewable form that then has an artificial flavoring added to cover the taste of the supplements.

The taste and consumption profile of these chewable tablets does not try to provide for a desirable consumption experience, but to disguise the "medicational" nature of the supplement in a form that is still essentially a tablet. Because of this, strong tasting artificial flavorings are commonly used and the structure is usually selected to provide for a minimal eating experience. In effect, the vitamins are simply made chewable to make them capable of being eaten by an individual who is likely going to be taste adverse to the straight supplement and is unable (or unwilling to attempt) to safely swallow a standard tablet.

Outside of chewable vitamin, a number of supplements have also tried to use food substrates as carriers. Many of these utilize placing the supplement into chocolate or into something that is effectively candy (e.g. a "gummy bear"). While this can make the supplement a desirable treat or snack and increase willingness to consume the supplement, in many cases the food substrate is detrimental to the user. In the case of such candies, the benefit of taking the supplement may be present, but consumption of these may have other negative effects (such as introduction of a large amount of refined sugar, fat, or caffeine).

Cinnamon and a number of other supplements (such as but not limited to ginger), can also have other significant problems in being used with the above food delivery systems. For one, they have very strong tastes and therefore when provided in a chewable form, the taste can overwhelm any flavoring designed to cover them up or provide for an unpleasant interactive taste. Further, cinnamon, in particular, has a benefit of having effects related directly to food consumption and digestion. This benefit is lost if the cinnamon is provided with too significant a food substance because the carrier, in effect, counteracts some or all of the cinnamon's benefit. Therefore, including cinnamon in a solid chocolate, high fat, or high carbohydrate carrier could, in fact, provide little to no benefit or even be detrimental to the desired purpose of taking cinnamon in the first place.

The issue with regards to providing relatively large quantities of a material which has a strong taste is not unique to cinnamon. A number of naturally occurring flavorings such as fruit extracts, fruit rinds, tree barks, and plant materials have also been found to have medicinal properties when consumed in larger quantities. However, like cinnamon, these have traditionally only been used as flavorings for foods that are relatively high in calories, fats, and/or sugars or have been provided in a pure medicinal form (such as a tablet) for consumption of larger quantities due to their taste. While many fruit flavors are pleasant, the need to use them in extracted or concentrated form to allow them to provide medicinal effect can often result in the taste being overpowering and unpleasant.

Based on the above, one should be able to see that the consumption of various materials has been limited to one of two forms. In the first, the material is provided as a flavoring, where its own taste characteristics are used to influence the taste of a food substance, but the principal purpose of the food substance is to provide calories (energy), as opposed to the nutritive benefit of the substance. Alternatively, the substance is provided in a medicinal, concentrated, form to provide medicinal benefit, but its natural taste is either concealed behind other flavorings, or the substance is intended to be consumed without tasting it.

### SUMMARY OF THE INVENTION

According to the present invention there is provided an edible composition of matter as set out in claim 1. Further features are set out in claim 2 - 14 to which attention is hereby directed.

For these and other reasons there are described herein systems and methods for providing a nutritional supplement in a food product. Specifically, a food product which is itself a desirable food to be eaten and tasted and which does not introduce significant calories or other materials which could counteract the benefit of the supplement. The food product is generally designed to utilize the natural flavor of the supplement to enhance the food as opposed to trying to cover it and providing for a pleasant eating experience to allow for the supplement to act not only as a supplement, but also as a food substitute. There is also described herein a food product produced by such methods and systems. In an embodiment, the food product is designed to use as a dessert replacement. That is, it is a food product which can provide for many of the expected characteristics of a dessert (e.g. sweeter tasting) while still providing a generally beneficial effect.

In an embodiment of the invention, the resultant product balances the additive ingredient with other ingredients so as to make the oral chewing and swallowing of a relatively large amount of additive a pleasing experience, while at the same time minimizing in the food product materials which would serve to counteract the additive's benefit to the consumer. In an embodiment, the taste characteristics of the multiple additives are balanced with each other. Specifically, the food carrier is designed to be relatively low calorie while still tasting pleasant (and preferably having a dessert type taste) and providing an enjoyable eating experience. The food product provides a medicinal dose of additive, while providing a sufficiently tasteful and complementary food product to the additive's taste along with keeping the detrimental health impact (that is total calories of consumption as well as consumption of negative food products such as fats and refined carbohydrates) of the food product to an acceptable low. In an embodiment, the taste of the additive is stronger than normally used for flavoring as an extract or concentrated version is used.

Though direct taste may be diluted via the chew process, there are significant other utilitarian benefits of actually chewing and tasting a physical product in one's mouth versus bypassing this process by swallowing a capsule or tablet whole. Food products of the invention also possess enough bulk that parts of the product is swallowed without chewing action being present on each bit of additive providing for consumption of a greater quantity without necessarily a corresponding increase in taste sensation. The food described herein, therefore, serves to meet the need of an after meal "sweet" or of a "sweet snack" while providing a healthful alternative to most traditional desserts. Those having such a post-meal dessert craving are often referred to, or refer to themselves, as having a "sweet tooth" satisfying such craving is generally a normal although often detrimental, human need.

Described herein, among other things is a composition of matter comprising: about 0.5 grams to about 3 grams of additive such as a fruit extract or cinnamon per 20 grams of total matter by weight; precooked grain; and a low-glycemic sugar or mostly low-glycemic sugar.

In an embodiment of the composition the precooked grain comprises wheat such as, but not limited to, crushed shredded wheat or crushed wheat flakes.

In an embodiment of the composition the precooked grain comprises dehydrated cooked rice.

In other embodiments of the composition both a fruit extract and cinnamon are included as additives. In these embodiments, the cinnamon comprises Ceylon cinnamon, cassia cinnamon, or any combination of the two.

In embodiment of the composition, the low-glycemic sugar comprises honey, such as, but not limited to, agave honey and/or comprises sugar alcohol such as, but not limited to, xylitol and/or erythritol.

In an embodiment, the composition also comprises, molasses, bee honey, additional dry sweetener, ground ginger root, sea salt, vanilla extract, vanilla powder, nutmeg, cloves, and/or brown sugar.

In embodiment of the food substance, the low-glycemic sugar comprises a sugar alcohol and/or a honey such as, but not limited to, agave honey.

### BRIEF DESCRIPTION OF THE DRAWINGS

FIG. 1 Provides a flowchart showing the steps of an embodiment for manufacturing a food-based delivery system.

### DESCRIPTION OF THE PREFERRED EMBODIMENT(S)

To-date consumers lack a consumption option for the purposes of consuming cinnamon and/or other natural occurring flavorings in quantities thought to be medicinal in a form that is palate pleasing (in terms of taste), interesting (i.e. in terms of a chew process), aromatic (i.e. in terms of a pleasing smell), convenient in terms of ease of quick use, ease of maintaining freshness for long periods and ease of daily availability in terms of keeping cinnamon on one's body for later consumption during the day. This can be especially true since for many additives, such as cinnamon, consumption during or after a meal is believed to be beneficial and is the recommended timing for consumption by many manufacturers. People consume many of their meals away from home or in other settings where adding an additive to the food/beverage they are consuming would require significant extra effort and likely provide for unpleasant taste interaction. Alternatively, the additive is taken in capsule form and therefore while it is "added" to the meal, it is not substituted for what may be a less healthy part of the meal.

Because of this, in its preferred form, the food substance is a ready-made, easy-to-carry, delivery method/product -- other than capsules/tablets which lack some of the consumer satisfying "eating characteristics" since they are swallowed whole. Attempting to chew such tablets or capsules generally produces an undesirable dry, bitter, and tart taste and chew process and would generally be actively discouraged.

This disclosure will discuss a product which is generally referred to as a supplement food product and a process and method for producing such a food product. The product is generally designed for consumption by humans and can be provided in a convenient to carry and consume ready-to-eat food substance incorporating a typical medicinal dose of cinnamon and/or other natural flavoring or medicinal item such as, but not limited to, a fruit extract, plant extract, tree bark, or related plant material and having positive palate ratings in terms of taste and chewing sensations relative to other ready-to-consume medicinal modalities. The food substance is also designed to not significantly negate the positive effects of the additive consumption, nor include any significant amount of ingredients known to be deleterious to good health if consumed in abundance, such as, but not limited to, fat, sugar, carbohydrates and sodium. In this way, the food product is designed to provide for health benefits for increased consumption without introducing major deleterious health effects from consumption of the food substance and can be substituted for other foods which traditionally include such materials. As such, in an embodiment, the food product can be used frequently (for example, on a daily or even multiple use per day regimen) for an indefinite, and potentially constant, period of time.

Part of the goal of providing such a substance is to provide that the medicinal effect is provided as part of an eating experience. Instead of the substance being consumed in a manner solely designed to provide for nutrition, the food substance provided herein is intended to provide for the additive in a format which is regarded as pleasing to the palate, and utilizes the substances natural flavor characteristics instead of hiding them. This is used in conjunction with providing a food substance which provides for pleasing texture and palatability without the inclusion of significant deleterious materials such as fat, saturated fat, and refined sugars.

It should be recognized that certain terms within this disclosure are intended to have certain meaning and are used specifically. This disclosure will discuss the cinnamon or other "additive" being provided as part of a foodstuff, food substrate, food product, food substance, or food. The term "food" is used herein to indicate that the carrier is intended to be chewed and tasted, as opposed to being swallowed whole (in the manner of a tablet) or drunk. A food will generally be chewed and therefore ground into smaller pieces in the act of chewing, before being swallowed. This will also result in the food being tasted when it is chewed by an ordinary human user. Further, a food goes beyond a simple product which is just designed to be chewed. The product is designed to act as a food substitute having pleasant taste, chewing characteristics, and sufficient mass to feel satisfying to a human user, so as to potentially work as an alternative to a less healthy snack or dessert.

This disclosure will also discuss the "additive" which is the material added to the food to provide it with desirable health characteristics. Generally, an additive, as contemplated herein, will be a powdered or liquefied form of a plant component or extract thought to have beneficial health effect when consumed in a medicinal quantity. Additives may comprise a variety of different materials, but in a preferred embodiment, will comprise a tree bark such as cinnamon. In alternative embodiments, the additive may comprise a fruit extract, such as, but not limited to, extracts of cherry, mulberry, boysenberry, gooseberry, black currant, red currant, tart cherry, black cherry, cherry acerola, West Indian cherry, tangerine, acai berry, goji berry/wolfberry, baobab, prickly pear, apple, cranberry, orange, lemon, lime, grapefruit, grapeseed, grape, banana, pomegranate, blueberry, guava, fig, apricot, mangosteen, mango, papaya, pineapple, strawberry, melon, red raspberry, or black raspberry. The additive also need not comprise a fruit extract, but may comprise a powdered or other fruit rind such as, but not limited to, that of lemon, orange (sweet or bitter), lime, tangerine, grapefruit, or melon. Many of these materials are high in antioxidants and other beneficial vitamins. Further, the additive in the food product will be intended to be tasted. That is, the flavor of the additive will enhance the food product, as opposed to being covered by other flavors.

This product will also discuss a "medicinal dose" of cinnamon or other additive. This term is not meant to imply that the amount specified serves to provide any specific health change or cure, prevent, or inhibit any disease or symptom. However, medicinal doses of cinnamon and/or other ingredients discussed herein can be used as an appetite suppressant. This is intended merely to distinguish the amount from that necessary to be used as a flavoring. A medicinal dose is therefore intended to show that the product includes more of an additive than is truly necessary to serve as a flavoring and that such an amount is currently believed, in light of current science, to provide for a health benefit. A medicinal dose of an additive will be considered an amount of at least 1 gram consumed in a single instance. Further, the food may be referred to as providing a supplement. This again is not specifically intended to refer to nutritional supplements as defined by the Food and Drug Administration (FDA) but to indicate that the additive is being provided in a level above that of a standard flavoring. The foodstuff discussed herein may, or may not, be classified as a nutritional supplement by the FDA. In some embodiments, the food may be used as a diet or weight maintenance aid with both the supplement serving to provide such benefit and the food serving as an acceptable substitute for a higher calorie food.

The one gram of additive may be provided in a single food substrate or may be provided on multiple substrates which are intended to act as a single "consumable dose." For example, the about 1 gram may be provided in a single food substrate bar, or may be provided across two substrate pieces, which, in combination, have similar weight to the bar. Generally, a serving of food substance will be an amount of between 16 and 35 grams, more preferably about 20-30 grams and still more preferably about 28-30 grams. This amount is generally about the normal serving size for packaged cookies or similar foods. Therefore, it would be generally preferred for a single gram of additive be provided in every 16-35 grams of food substance. It should be recognized, however, that the concept of a consumable dose does not require that that specific consumable dose be consumed in any sitting or over any time period. For that reason the ratio of about 1 gram of additive may be present in every 16-35 grams of food substance, regardless of the amount of food substance which is to be consumed at any one time, in any time period, or in any one form as provided to a consumer. Further, a single food substance need not only include a single additive. In an embodiment, the food will include medicinal doses of a plurality of different additives and, therefore, the total percentage of additives will be greater.

As should be apparent from the above, a consumable dose refers to the amount of food substrate that the user is intended to consume in a single instance. As the food substrate provided herein is designed, in an embodiment, to act as a dessert substitute, the food substrate will generally be in the form of one to seven small biscuits, cookies, or bars which are intended to be consumed together and therefore comprise a consumable dose. In an alternative embodiment, the dose may be from two to three objects. Further, the food substrate, in this way, can act as a substitute for alternative desserts and be consumed immediately following a meal (as is often recommended to gain medicinal effect from cinnamon and many antioxidants which are often plentiful in fruit materials). The use of multiple pieces in the single serving is preferred as it can make the consumption a longer task and the food substance to appear more substantial than if it was in a single piece. Further, the cookie form can provide for desirable substitution of the supplement and food substance for a food which generally has minimal health benefits other than as a source of calories. Still further, using an increased number of smaller pieces can provide that the consumption is more satisfying.

In an alternative embodiment, the food substance can be assembled, but then be crumbled, crushed, or powdered and added to other food items (such as, but not limited to, acting as a topping for yogurt or salad) to provide for the same ratio of additive to food substance ingredients. This method is generally not preferred at this time due to the potential introduction of extra calories from the underlying consumable on which the food substance is placed and the fact that the food is not being used as a substitute. However, for consumption of the product for certain individuals it may be desirable and provides for an alternative manner of consumption of the product.

The food item, as contemplated in an embodiment, comprises a cooked biscuit or "cookie" that is designed to provide for a medicinal dose of cinnamon and/or other additive, while at the same time avoiding the inclusion of undesirable food elements such as excess fat, excess simple carbohydrates, or unnecessary calories. It is further desired that the cookie be designed to be shelf stable under standard conditions for a relatively long period of time. In this way the term "cookie" is a bit of a misnomer because the food substance described herein does not include many of the hallmark ingredients of a cookie. However, as the food substance is designed to be eaten after a meal or as a satisfying snack, which can be used to curb future hunger and improve blood sugar load, reference to a dessert item such as a cookie is logical.

The cookie will generally include a medicinal amount of additive. Generally, the amount will be selected to have about between 0.75 and about 1.5 grams of each additive per serving with the idea of providing at least 1 gram of each additive in every consumable dose with a consumable dose comprising one to seven cookies and preferably either three larger cookies or six smaller (half-size) ones. Again, the preferred ratio of additive is about 1 gram to about 16 to about 35 grams of food substance.

When cinnamon is used, the cinnamon included may be any combination of available cinnamons but it is preferred that the resultant cinnamon comprise about 50% or more Ceylon cinnamon. Ceylon cinnamon has a generally milder taste than more common cassia cinnamon. By keeping the percentage of Ceylon cinnamon higher, the resultant taste is often more pleasant. However, cassia cinnamon is generally significantly cheaper to produce and may include unique health benefits not present in Ceylon cinnamon. Therefore, the two may be intermixed to provide for a more cost effective production scheme, a different supplement profile, and a slightly different taste. Further, it should be recognized that while use of a mixture of cinnamons is preferred, it is by no means required and the cookie may be made with 100% cassia or 100% Ceylon cinnamon or with any other cinnamon or combination of cinnamons which may or may not include either of the above.

When a fruit additive is used it is generally preferred that the additive be a fruit extract or fruit rind eliminating much of the sugar and other fillers (such as water) in the fruit as possible, and maximizing ingredients thought to provide health benefit. In such an embodiment, each additive will generally comprise more than 2% of the resulting food substance. In an embodiment it will comprise from about 2% to about 15% of the resulting food substance by weight. In another embodiment from about 2% to about 7% of the resulting substance. However, it is generally preferred that any one additive comprise from about 3% to about 5% by weight. In an embodiment, at least one gram of fruit extract will be present in a single serving size. In the cookie as a whole when multiple additives are used, the additives will usually total more than 3% by weight, more than 10% by weight or more than 15% by weight. In an embodiment, an amount between 10% and 15% is desired as it provides a relatively large quantity of additive, without burying the food substrate characteristics with the additive and providing an overpowering taste profile.

As a part of the food substance discussed herein is to provide for a pleasant taste characteristic and eating sensation, it should be recognized that the inclusion of two much of any additive, or a mixing of additives with undesirable interacting taste characteristics, would generally produce an undesirable net result. Therefore, the food substance will generally utilize three main components to produce a desirable taste and texture. The additive (or additives) will serve to provide for principal flavoring with the composition being designed so that the flavoring characteristics of the additive are not concealed, but instead provide for a desirable flavoring of the present composition. Often, a "sweet" additive will be combined with a "bitter" or citrus additive to provide a desirable overall taste profile. The second component will generally be that of precooked grain which is principally intended to provide for mass and texture to the food. The final material will usually be a sugar or other binder. As opposed to dough and similar compositions where sugar is used as a sweetener, or as opposed to candies where sugar provides for the principal crystalline structure of the composition, the sugars in an embodiment may serve to act as binders for the other materials to allow for the composition to retain its shape after cooking.

While there are a great many compositions of additives which can be used in different embodiments of cookies as described herein, one preferred embodiment comprises (per 30 gram serving): about 1 gram cinnamon (50/50 cassia and Ceylon), about 1 gram sweet orange peel, about 1 gram tart cherry extract, and about 1 gram goji berry extract. This embodiment provides for a cookie having more than 10% of it total mass by weight being from additives with each additive totaling about 3% of the total mass. It provides a taste profile which is balanced and interesting, and does not provide an overpowering taste. It should be noted that many commercially available fruit extracts and fruit rind compositions include fillers or non-active elements (e.g. fiber or water). The above ratios generally presume that the extract comprises about some percentage of its total weight from fillers and therefore the actual medicinal elements may comprise only a portion of total listed weight. In an embodiment the extracts or rinds are provided in about 4:1 commercial concentration. However, in an alternative embodiment purer or less refined materials may be used allowing for slight alterations in the relative weights. In a still further embodiment, the measure may refer to actual extract or rind weight or to weight of specific elements considered active in such specific fruits or rinds.

The food substrate into which the additive is added will generally include a number of other ingredients. As the substance is designed to be a food which is eaten as a dessert substitute, it is desirable for the substance to be able to provide a 16-35 gram serving size common in desserts. Again, it is important to recognize that the food substrate will generally not comprise the hallmark ingredients of cookies (e.g. eggs, butter or similar oils, or milk), but will utilize grains to provide for texture, and sugars to act as binders. As many of the additional ingredients are not used, it is possible, in an embodiment, for the resulting food product to be considered "vegan."

In order to provide mass and texture, the food substrate will generally include various grains which are generally crushed or pulverized. The exact size of the resultant particulate material is not specifically required, but in a preferred embodiment the grains will generally be larger than about 0.5mm in major dimension (about the size of a grain of salt) and smaller than about 7mm in major dimension (about the size of a grain of rice). However, at least a portion of the grain is preferably a flour and often a gluten free flour (such as a bean flour) because it is believed that inclusion of such a material can assist with the binding. Use of a gluten free flour is generally preferred as it can result in a stiffer and chewier product. Since the product generally does not utilize a leavener, although it may include a yeast such as, but not limited to, nutritional yeast flakes or brewers yeast, there is no need to utilize gluten to capture air in the product. Flours will generally have a grain size smaller than crushed particulate grains, but that is by no means required or necessary. It is also generally preferred that fat free flours be used.

In an embodiment, it is preferred that the grain mix include mostly or exclusively grains which are cooked prior to addition to the food substrate mixture as this can improve shelf life and texture. Precooked flours may be used, however, in one embodiment, other precooked grains and grain mixtures (such as cereals) may be crushed and utilized. In an embodiment, the principal grain will be wheat since it generally is shelf stable once cooked however grains such as oats, corn, rice, barley, millet, flax, hemp, or other grains may be included or substituted. Further, it is generally preferred that a mixture of grains and/or grain forms be used and not a singular grain form alone. Use of a single grain can result in the product having an undesirable texture which is more uniform and lacks distinctiveness and which provides a less pleasant eating experience than if a variety of grain forms are used.

In an embodiment, the same grain can be used in multiple forms. Specifically, wheat can be partially supplied in a shredded or ribbon form (such as in shredded wheat cereals) which is crushed after cooking and partially in flake (molded) form (such as in crushed Wheaties™ and Weetabix™ cereals) which is crushed after cooking. These can be combined as the forms generally have different textures. Bolger wheat can also be included or substituted with other wheat ingredients in another embodiment.

In a still further embodiment, the cookie includes precooked dehydrated rice as a further grain. This type of product is commonly sold as Minute Rice™ or Instant Rice™ and comprises rice which has been cooked and then dehydrated. The rice is generally not rehydrated prior to being placed in the mixture but is placed in dry form where it provides a slightly chewy texture.

There will generally also include a binder and sweetener. So as to avoid refined white sugar and the attendant caloric and glycemic load, it is preferred that low-glycemic sugars be used. In an embodiment, these include sugar alcohols (or polyols) such as xylitol, erythritol, or sorbitol, or may include stevia (aka sweetleaf or sugarleaf). In another embodiment, low glycemic sugars such as honey may be used as a low glycemic sugar. These low glycemic sugars are generally used in combination. It should be recognized that it is not necessary for the resultant food to be low glycemic, but it is generally preferred that excess or unnecessary glycemic load is avoided. In a still further embodiment, plant nectars (e.g. Agave Honey) may be used as sweeteners.

Sugar alcohols are generally preferred to be included (either alone or in combination) since they are generally considered to have little to no effect on blood sugar or insulin levels. As such, their inclusion provides for sweeteners and therefore improved taste without serving to increase blood sugar and therefore potentially cancel out the expected benefit of blood sugar regulation from the cinnamon intake.

In an embodiment, the sugar alcohols are provided crystallized as dry sweeteners. However, they may be used in liquid form in an embodiment. In an embodiment, liquid sweeteners such as Agave nectar and molasses also act as a binder. In alternative embodiments, the dry sweetener may be removed and additional wet sweetener may be used or vice versa. Further, other sugar alcohols or sugars may be added in a liquid or crystallized form.

The sweetener is preferably a combination of Agave honey and molasses although other sweeteners or combinations may be used. Agave honey and many similar sweeteners are desirable to be used as they also provide for a relatively low glycemic load and therefore may be considered low-glycemic sugars. Alternative sweeteners such as Agave honey alone or in combination with sugar alcohols are often preferred as consumption of large quantities of sugar alcohol has been known to cause diarrhea and gas. Therefore, it is preferable that the product have less than 15% sugar alcohol by mass and more preferable that it have less than 10% sugar alcohol by mass.

The binders are generally preferred to be heat activated and serve to alter their structure once exposed to heat. These are often sugars which can change crystalline structure after being heated. The food substance will generally not use binders that work best at room temperature since those are often insufficient to create a desirable resultant texture. Further, it is generally preferred that the sugar binders utilize their own alteration in crystal structure to serve to lightly bind, as opposed to utilizing fats or other materials to interfere with crystallization to provide the same type of binding characteristics. For this reason simpler sugars (such as fructose and glucose) may be preferred to sucrose however, high fructose corn syrup may be undesirable due to its possible connection with carbonyl compounds. Use of sucrose may similarly be preferred with an inverter to break down the sucrose into components and prevent hard crystallization.

As contemplated above, some of the sweeteners will comprise liquids and some solids. It is generally preferred that about 25% to about 40% of the total ingredients (by mass) comprise liquids to provide for a resultant product which is moldable. In an embodiment, it is preferred that about 34% of the materials be liquid with the rest being solid. This is generally believed to be a lower percentage of liquids than is common in many cookie mixtures which results in a mixed dough which is more crumbly until being heated.

Generally, it is preferred that the resultant mixture, prior to heating, not comprise a "dough" or other material of similar composition. Dough is traditionally recognized as a mixture of a grain meal and water which produces a material which can be molded, rolled, or otherwise easily manipulated. Instead, the material is generally provided as a loose granular mixture which requires pressure to form into shape. Such a mixture is generally preferred as it allows for the mixture to be focused on generally solid ground ingredients with less need for inclusion of water, proteins, or leaveners.

As is understood by those in the art, materials which lack sufficient liquid may not suitably bind, even when exposed to heat, which can result in the cookie being overly crumbly, therefore reducing the liquids to below 25% is generally undesired. At the same time, increasing the percentage of "wet" ingredients (that is, ingredients including water) is generally undesirable as it makes the resulting product also lack structure since the product will generally lack the chain proteins provided by eggs or similar ingredients. It is generally desirable, to maintain a relatively low glycemic load and enhance the effectiveness of the cinnamon, that the food substance comprise less than about 25% sugar with less than about 10% sugar alcohol with around 20-40% of the sugar being sugar alcohols.

In addition to the above, a number of flavoring ingredients may also be included to provide for improved flavoring. As should be apparent, the principal flavoring is the additive or additives themselves and therefore additional flavorings are generally provided which complement the flavor of the selected additive(s). As opposed to the additive, however, these are not added in medicinal dose quantities and are provided solely for flavoring. Generally, there will be at least 3 times the amount of additive included as there would be of any flavoring but this is by no means required. Some flavorings which can be used are vanilla extract, vanilla powder, nutmeg, cloves, salt, brown sugar, ginger, chocolate, mint, or any combination of these. However, none of these are required to be included.

The resultant product is generally produced in an embodiment in the following manner as shown by the flowchart of FIG. 1. Initially, cereals and other dry products are crushed to provide for particles of desired size (101) as discussed above. As discussed above, most if not all of the grain ingredients will be precooked prior to this step.

Cinnamon is also generally crushed or ground into a fine powder form in step (103) as is standard for ground cinnamon. However, it is preferred that the cinnamon be ground non-uniformly allowing for larger pieces to be present. In this way the flavor may be decreased across the food as a whole when compared to using finely ground cinnamon. However, larger pieces may also provide for a distinctive taste sensation when the person actually crushes one of the larger pieces by the chewing action. It is believed this can provide for unique taste without making the cinnamon taste overly strong. Fruit extracts and rinds will also preferably be in powder form. Once the dry products are all at an acceptable size, they are mixed together with other dry ingredients including the dry sweeteners and any dry flavorings which are being included.

Liquid materials are mixed together in step (105) along with uncooked gluten-free flour. However, in alternative embodiments, the flour may be added to the dry ingredients as may some of the dry ingredients such as the flavorings or these may be added to the liquid ingredients to provide more uniform distribution. Adding these to the liquid mixture can assist in making sure they are more evenly distributed throughout the resulting mixture. It may be necessary to heat the liquid mixture (107) at this stage to provide for a sufficient fluid state to allow for intermixing with dry ingredients although, depending on the exact ingredients used, this may not be necessary. The liquid materials should have a generally fluidic state prior to continuing.

The dry materials are then added to the liquid materials in step (109) to form a generally uniformly dispersed mixture. The mixing may be performed at room temperature or slight heat may be added to maintain a fluidic viscosity to allow the material to be stirred relatively easily and form a fairly uniform dispersed mixture. Inclusion of fruit extracts is generally preferred with the dry ingredients as such extracts are preferably provided as powders to reduce the amount of fillers included with the extracts and better provide for a controlled amount of the extracts.

The mixture is then molded into a desired shape and size in step (111). As discussed above, it is generally preferred that the food substrate be formed into cookies or biscuits with two to six of such comprising an intended serving. Such formation can be based simply on making sure that the desired amount of additive is expected to be present in each subdivision of the mixture. So, for example, if the mixture includes 10 grams of cinnamon uniformly distributed, one would expect that the mixture would be formed into 20 cookies so as to have about 0.5 grams of cinnamon per cookie. However, other methods may be used as is understood by those of ordinary skill. Ingredients are generally selected so as to provide each resultant cookie to have a total weight of about 3-24 grams at precooked levels, generally around 5-10 grams, and preferably around 5 grams. Therefore, each additive generally comprises around 2.5% or more of the total weight of the cookie, an amount far exceeding what is traditionally used as a flavoring. Each cookie will generally comprise sufficient sweetener to bind, with the remaining composition comprising mostly grains with relatively trace amounts of flavorings and incidental materials (such as residual water).

Shape of the cookies is irrelevant and any conventional shape may be used. In an embodiment they may be formed into flat rounds or half domes which are standard cookie shapes. However, in alternative embodiments they may be formed into bars. In a still further embodiment, to assist with mechanical packaging, the mixture may be baked in a generally continuous flat sheet which is cut into desired shapes after baking.

It should be recognized that in an embodiment, the cookies may be formed under significant pressure. In an embodiment, the pressure is sufficient to provide for binding of the mixture together and the baking step may be eliminated completely. This is generally not preferred, however, and generally the pressure is simply sufficient to provide for the mixture to have a sufficiently rigid structure to survive transfer to the baking process without breaking apart. In an embodiment, the composition could be described as a conglomerate or as a wet granular pack and may be relatively fragile pre-baking.

Once the mixture is arranged in the desired form for baking, it is generally baked in step (113) if baking is desirable for binding. As the raw ingredients do not include any which generally must be cooked to provide for elimination of potentially harmful bacteria (such as eggs) assuming sufficient cleanliness is maintained in the assembly process, the principal purpose of baking is to activate binders by the application of heat. For this reason the baking process may be insufficient to sterilize the products or to "cook" any ingredients.

In an embodiment, the heating is at relatively low temperature, such as between 338K and 422K (150 and 300 degrees Fahrenheit), more preferable around 394K (250 degrees Fahrenheit). Heating occurs for around 5-30 minutes, more preferably about 5-15 minutes, and more preferably about 10 minutes at about 394K (250 degrees Fahrenheit). The resultant cookie in this cooking scheme has a moister texture while still having a relatively stiff exterior to provide for strength and binding and to inhibit breakage in transport and crumbling when being handled. However, in alternative embodiments a wide variety of baking values can be used ranging from about 30 minutes at about 338K (150 degrees) to about 5 minutes at about 422K (300 degrees). In still further alternative embodiments, the mixture may be heated at much higher temperatures (e.g. those above 478K or 400 degrees Fahrenheit) for much shorter periods of time. The exact cooking time and temperature depends on the desired resultant texture with lower temperatures generally providing a moister product and higher temperatures providing a crispier product. Further, if the food substance is designed to be crumbled, it may be baked longer to allow for a drier product more easily crumbled either mechanically or by hand.

Once baking is complete, cookies are generally allowed to sit and cool at about standard room temperature and humidity to allow them to absorb environmental moisture for about 12-48 hours (115). As opposed to many traditional cookies, where being served above room temperature can enhance texture by weakening binders (making them "soft" or "gooey"). These cookies generally appear to benefit from being allowed to stabilize at room temperature and humidity. Alternatively, similar resultant exposure under different environmental conditions may be provided to have the same result. The cookies are then generally packaged in airtight containers, possibly with nitrogen gas being infused in packaging, to inhibit any spoilage and provide protection for transportation.

While airtight packaging is desired, it should be recognized that the preferred ingredients used are generally only minimally responsive to spoiling and rancidity even without refrigeration or other preservation. Cinnamon itself also can serve to act as a preservative for the cookies. Therefore, airtight packaging is mostly preferred to prevent outside contamination from being introduced to the cookie, to provide for enhanced shelf life, to provide ruggedness for transportation, and to improve product appearance.

## Claims

1. An edible composition of matter for consumption by a human through chewing and tasting, which edible composition comprises:
0.5 grams to 3 grams of cinnamon per 20 grams of total matter by weight being a quantity sufficient to impart an unpleasant taste with a bitterness or tartness whilst the composition is chewed and tasted by the human;
a pre-cooked grain; and
a binder and sweetener, the binder and sweetener in the form of a low-glycemic sugar, the low-glycemic sugar being less than about 25% of the edible composition, whereby the quantity of low-glycemic sugar is sufficient to bind the cinnamon and pre-cooked grain, and sufficient to sweeten the composition just enough to restore a pleasant taste to the composition during chewing, but not a quantity that would counteract the benefit of the cinnamon to the human.

2. The composition of claim 1 wherein said pre-cooked grain comprises wheat, such as Bulgur wheat, crushed shredded wheat and/or crushed wheat flakes.

3. The composition of claim 1 or 2 wherein said cinnamon comprises Ceylon cinnamon and/or cassia cinnamon, optionally in approximately equal ratio.

4. The composition of claim 1, 2, or 3 wherein said binder and sweetener further comprises molasses.

5. The composition of any preceding claim wherein said low-glycemic sugar comprises a plant nectar, such as agave honey, and/or bee honey.

6. The composition of any preceding claim, wherein said low-glycemic sugar comprises sugar alcohol, such as at least one sugar alcohol selected from the group consisting of sorbitol, xylitol and erythritol.

7. The composition of any preceding claim, further comprising at least one flavoring selected from the group consisting of: ground ginger root, sea salt, vanilla extract, vanilla powder, nutmeg, and cloves.

8. The composition of any preceding claim, further comprising a fruit extract.

9. The composition of claim 8, wherein said fruit extract serves to provide the principal flavoring of the composition.

10. The composition of any preceding claim, wherein said composition is in the form of a food product, and optionally the food product is designed for consumption by humans, and optionally is in a convenient to carry and consume ready-to-eat food substance.

11. The composition as claimed in claim 10, wherein the food product comprises a cooked biscuit.

12. The composition of claim 6, wherein said sugar alcohol is less than about 10% of the edible composition.

13. The composition of claim 12, wherein said sugar alcohol is 5% to 10% of the edible composition.

14. The composition as claimed in any preceding claim, having a weight of between 10 to 18 grams.

## Patentansprüche

1. Essbare Stoffzusammensetzung zum Verzehr durch einen Menschen durch Kauen und Schmecken, wobei die essbare Zusammensetzung umfasst:
0.5 g bis 3 g Zimt pro 20 g Gesamtmasse, was eine Menge ist, die ausreicht, um einen unangenehmen Geschmack mit einer Bitterkeit oder Säure zu verleihen, während die Zusammensetzung von dem Menschen gekaut und geschmeckt wird;
ein vorgekochtes Getreide; und
ein Bindemittel und Süßstoff, wobei das Bindemittel und der Süßstoff in Form eines niederglykämischen Zuckers vorliegen, wobei der niederglykämische Zucker weniger als etwa 25% der essbaren Zusammensetzung beträgt, wodurch die Menge an niederglykämischem Zucker ausreicht, um den Zimt und das vorgekochte Getreide zu binden, und ausreicht, um die Zusammensetzung gerade genug zu süßen, um der Zusammensetzung beim Kauen einen angenehmen Geschmack zurückzugeben, aber nicht eine Menge ist, die dem Nutzen des Zimts für den Menschen entgegenwirken würde.

2. Zusammensetzung nach Anspruch 1, wobei das vorgekochte Getreide Weizen, wie beispielsweise Bulgur-Weizen, gemahlenen Weizenschrot und/oder gemahlene Weizenflocken umfasst.

3. Zusammensetzung nach Anspruch 1 oder 2, wobei der Zimt Ceylonzimt und/oder Cassiazimt umfasst, wahlweise in etwa gleichem Verhältnis.

4. Zusammensetzung nach Anspruch 1, 2 oder 3, wobei das Bindemittel und der Süßstoff des Weiteren Melasse umfassen.

5. Zusammensetzung nach einem der vorangegangenen Ansprüche, wobei der niedrigglykämische Zucker einen pflanzlichen Nektar, wie beispielsweise Agavenhonig, und/oder Bienenhonig umfasst.

6. Zusammensetzung nach einem der vorangegangenen Ansprüche, wobei der niedrigglykämische Zucker Zuckeralkohol umfasst, wie beispielsweise mindestens einen Zuckeralkohol, der aus der Gruppe bestehend aus Sorbit, Xylit und Erythrit ausgewählt ist.

7. Zusammensetzung nach einem der vorangegangenen Ansprüche, des Weiteren umfassend zumindest ein Gewürz, das ausgewählt ist aus der Gruppe bestehend aus: gemahlener Ingwerwurzel, Meersalz, Vanilleextrakt, Vanillepulver, Muskat und Nelken.

8. Zusammensetzung nach einem der vorangegangenen Ansprüche, des Weiteren einen Fruchtextrakt umfassend.

9. Zusammensetzung nach Anspruch 8, wobei der Fruchtextrakt dazu dient, das Hauptaroma der Zusammensetzung bereitzustellen.

10. Zusammensetzung nach einem der vorangegangenen Ansprüche, wobei die Zusammensetzung in Form eines Lebensmittelprodukts vorliegt und wahlweise das Lebensmittelprodukt für den menschlichen Verzehr bestimmt ist und wahlweise in einer zum Mitführen und Verzehren praktischen verzehrbereiten Lebensmittelsubstanz vorliegt.

11. Zusammensetzung nach Anspruch 10, wobei das Lebensmittelprodukt ein gekochtes Biskuit umfasst.

12. Zusammensetzung nach Anspruch 6, wobei der Zuckeralkohol weniger als etwa 10% der essbaren Zusammensetzung beträgt.

13. Zusammensetzung nach Anspruch 12, wobei der Zuckeralkohol 5% bis 10% der essbaren Zusammensetzung ausmacht.

14. Zusammensetzung gemäß einem der vorangegangenen Ansprüche, ein Gewicht zwischen 10 und 18 Gramm aufweisend.

## Revendications

1. Composition de matière comestible pour consommation par un être humain par mâchage et dégustation, laquelle composition comestible comprend :
0,5 gramme à 3 grammes de cannelle pour 20 grammes de matière totale en poids, qui est une quantité suffisante pour communiquer un goût désagréable avec amertume ou âpreté pendant que la composition est mâchée et dégustée par l'être humain ;
une céréale précuite ; et
un liant et un édulcorant, le liant et l'édulcorant se présentant sous la forme d'un sucre de faible indice glycémique, le sucre de faible indice glycémique étant de moins d'environ 25 % de la composition comestible, moyennant quoi la quantité du sucre de faible indice glycémique est suffisante pour lier la cannelle et la céréale précuite, et suffisante pour édulcorer la composition juste assez pour rétablir un goût agréable à la composition pendant le mâchage, mais non pas une quantité qui contrebalancerait le bénéfice de la cannelle apportée à l'être humain.

2. Composition selon la revendication 1, dans laquelle ladite céréale précuite comprend du blé, tel que du blé boulgour, du blé filamenté concassé et/ou des flocons de blé concassé.

3. Composition selon la revendication 1 ou 2, dans laquelle ladite cannelle comprend de la cannelle de Ceylon et/ou de la cannelle de Chine, facultativement dans un rapport approximativement égal.

4. Composition selon la revendication 1, 2, ou 3, dans laquelle ledit liant et ledit édulcorant comprennent en outre de la mélasse.

5. Composition selon une quelconque revendication précédente, dans laquelle ledit sucre de faible indice glycémique comprend un nectar de plante, tel que du miel d'agave, et/ou du miel d'abeille.

6. Composition selon une quelconque revendication précédente, dans laquelle ledit sucre de faible indice glycémique comprend de l'alcool de sucre, tel qu'au moins un alcool de sucre choisi dans le groupe consistant en le sorbitol, le xylitol et l'érythritol.

7. Composition selon une quelconque revendication précédente, comprenant en outre au moins un aromatisant choisi dans le groupe consistant en : de la racine de gingembre moulue, du sel marin, un extrait de vanille, de la poudre de vanille, de la muscade, et des clous de girofle.

8. Composition selon une quelconque revendication précédente, comprenant en outre un extrait de fruit.

9. Composition selon la revendication 8, dans laquelle ledit extrait de fruit sert à apporter l'aromatisation principale de la composition.

10. Composition selon une quelconque revendication précédente, dans laquelle ladite composition se présente sous la forme d'un produit alimentaire, et facultativement le produit alimentaire est conçu pour une consommation par des êtres humains, et facultativement se trouve dans une substance alimentaire prête à manger commode à porter et à consommer.

11. Composition selon la revendication 10, dans laquelle le produit alimentaire comprend un gâteau cuit.

12. Composition selon la revendication 6, dans laquelle ledit alcool de sucre est de moins d'environ 10 % de la composition comestible.

13. Composition selon la revendication 12, dans laquelle ledit alcool de sucre est de 5 % à 10 % de la composition comestible.

14. Composition selon une quelconque revendication précédente, ayant un poids compris entre 10 et 18 grammes.
